# EUROPEAN PATENT SPECIFICATION

(11) **EP 4 284 270 B1**
(45) Date of publication and mention of the grant of the patent: **04.06.2025**
(21) Application number: 22704687.7
(22) Date of filing: 28.01.2022
(51) Int. Cl.: A61B 17/34, A61B 90/00

(54) **HUMERAL INTRAOSSEOUS LANDMARKING APPARATUS**
INTRAOSSÄRE HUMERUS-LANDMARKIERVORRICHTUNG
APPAREIL DE MARQUAGE INTRA-OSSEUX HUMÉRAL

(30) Priority: 01.02.2021 US 202163144369 P
(43) Date of publication of application: 06.12.2023
(73) Proprietor: Bard Access Systems, Inc., Salt Lake City, UT 84116 (US)
(72) Inventor: PETT, Daniel, Sandy, UT 84093 (US); WARE, Thomas, D., Salt Lake City, UT 84115 (US)
(74) Representative: Hoffmann Eitle
(86) International application number: PCT/US2022/014391
(87) International publication number: WO 2022/165232

(56) References cited:
- WO-A1-2021/016122
- US-A1- 2015 045 732
- US-A1- 2017 020 560

## Description

### PRIORITY

This application claims the benefit of priority to U.S. Provisional Application No. 63/144,369, filed February 1, 2021.

### BACKGROUND

WO 2021/016122 A1 and US 2017/020560 A1 disclose landmarking devices for intraosseous injection needles.

Some current practices for humeral intraosseous needle placement involve manual tactile land marking over the lateral upper arm to determine proper intraosseous needle insertion site. For proper humeral intraosseous needle placement, the intraosseous needle should be inserted into the humeral head. For patients with thick musculature or thick adipose tissue within this region, tactile land marking to identify the humeral head can be difficult and time consuming. Furthermore, tactile land marking can be difficult for new or infrequent intraosseous needle placement users or in critical time periods. It would be beneficial to the clinicians to be able to consistently, and with minimal effort, correctly identify the humeral head through landmarking the humeral region for proper intraosseous needle placement. Disclosed herein is an apparatus and a method that addresses the foregoing.

### SUMMARY

A humeral intraosseous landmarking apparatus according to the invention is defined in claim 1. The dependent claims define preferred embodiments. Methods as such are not claimed.

Disclosed herein is humeral intraosseous landmarking apparatus having a tab including an acromion placement circle, a band including a lateral epicondyle placement circle, where the lateral epicondyle placement circle and the acromion placement circle are separated by a fixed landmarking distance, and an intraosseous needle ring configured to landmark a humeral head, the intraosseous needle ring having a proximal end coupled to the tab and a distal end coupled to the band.

In some embodiments, the apparatus includes a top side and a bottom side.

In some embodiments, the tab includes a tab length and the band includes a band length and the length is greater than the tab length.

In some embodiments, the lateral epicondyle placement circle is located at a distal end of the band.

In some embodiments, the tab, the band and the intraosseous needle ring include an adhesive compound on the bottom side.

In some embodiments, the intraosseous needle ring includes a beveled edge, where the beveled edge includes an angle within the range of 75°-110° in relation to the bottom side.

In some embodiments, the intraosseous needle ring includes an antimicrobial patch detachable coupled to the bottom side of the intraosseous needle ring.

In some embodiments, the apparatus includes a needle stabilizing device configured to fit within the intraosseous needle ring.

In some embodiments, the needle stabilizing device includes a needle stabilizing device lumen configured to receive an intraosseous needle and stabilize the intraosseous needle.

In some embodiments, the tab and the band include pressure paper configured to change from a first color to a second color with an increase in pressure upon the pressure paper by a hard surface.

In some embodiments, the tab includes a first score line configured to detach the tab from the proximal end of the intraosseous needle ring and the band includes a second score line configured to detach the band from distal end of the intraosseous needle ring.

Also disclosed herein is a method for landmarking the humeral region for intraosseous access, including configuring an arm of a patient for intraosseous needle placement, locating an acromion of the patient, overlaying an acromion placement circle of the intraosseous humeral landmarking apparatus over the acromion, locating a lateral epicondyle of the patient, overlaying the lateral epicondyle placement circle of the intraosseous humeral landmarking apparatus onto the lateral epicondyle, and inserting an intraosseous needle within an intraosseous needle ring of the intraosseous humeral landmarking apparatus.

In some embodiments, the intraosseous humeral landmarking apparatus includes a tab having the acromion placement circle , a band having a lateral epicondyle placement circle, where the lateral placement circle and the acromion placement circle are separated by a fixed landmarking distance, and the intraosseous needle ring configured to landmark a humeral head, the intraosseous needle ring having a proximal end coupled to the tab and a distal end coupled to the band.

In some embodiments, the intraosseous humeral landmarking apparatus includes a top side and a bottom side, where the bottom side includes an adhesive compound thereon.

In some embodiments, configuring includes adducting the patient's arm and configuring the patient's palm to be placed over a patient's umbilicus.

In some embodiments, locating the acromion includes using palpation, ultrasound imaging or infrared imaging for locating the acromion.

In some embodiments, overlaying includes adhering the acromion placement circle to the skin above the acromion.

In some embodiments, locating the lateral epicondyle includes using palpation, ultrasound imaging or infrared imaging for locating the lateral epicondyle.

In some embodiments, overlaying includes adhering the lateral epicondyle placement circle to the skin above the lateral epicondyle.

In some embodiments, inserting an intraosseous needle ring includes inserting an intraosseous needle within the intraosseous needle ring into a humeral head.

These and other features of the concepts provided herein will become more apparent to those of skill in the art in view of the accompanying drawings and following description, which describe particular embodiments of such concepts in greater detail.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 illustrates a perspective view of an arm including a humeral head.
FIG. 2 illustrates a plan view of the intraosseous humeral land marking apparatus, in accordance with some embodiments.
FIG. 3A illustrates a side cross sectional view of the apparatus including the intraosseous needle ring, in accordance with some embodiments.
FIG. 3B illustrates a perspective view of the intraosseous humeral landmarking apparatus on an arm, in accordance with some embodiments.
FIGS. 4A-4D illustrate an exemplary method of landmarking the humeral region for proper intraosseous needle placement, in accordance with some embodiments.
FIGS. 5A-5B illustrate a perspective view of an embodiment of the intraosseous humeral landmarking apparatus, in accordance with some embodiments.
FIGS. 5C-5E illustrate an exemplary method of landmarking the humeral region for proper intraosseous needle placement, in accordance with some embodiments.
FIG. 6 illustrates a flow chart of the exemplary method of land marking the humeral region using the intraosseous humeral land marking apparatus, in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "top," a "top portion" or a "top-end portion" of, for example, an apparatus disclosed herein includes a portion of the apparatus intended to be near a clinician when the apparatus is used on a patient. Likewise, a "top length" of, for example, the apparatus includes a length of the apparatus intended to be near the clinician when the apparatus is used on the patient. A "top end" of, for example, the apparatus includes an end of the apparatus intended to be near the clinician when the apparatus is used on the patient. The top portion, the top-end portion, or the top length of the apparatus can include the top end of the apparatus; however, the top portion, the top-end portion, or the top length of the apparatus need not include the top end of the apparatus. That is, unless context suggests otherwise, the top portion, the top-end portion, or the top length of the apparatus is not a terminal portion or terminal length of the apparatus.

With respect to "bottom," a "bottom portion" or a "bottom-end portion" of, for example, an apparatus disclosed herein includes a portion of the apparatus intended to be near or in a patient when the apparatus is used on the patient. Likewise, a "bottom length" of, for example, the apparatus includes a length of the apparatus intended to be near or in the patient when the apparatus is used on the patient. A "bottom end" of, for example, the apparatus includes an end of the apparatus intended to be near or in the patient when the apparatus is used on the patient. The bottom portion, the bottom-end portion, or the bottom length of the apparatus can include the bottom end of the apparatus; however, the bottom portion, the bottom-end portion, or the bottom length of the apparatus need not include the bottom end of the apparatus. That is, unless context suggests otherwise, the bottom portion, the bottom-end portion, or the bottom length of the apparatus is not a terminal portion or terminal length of the apparatus.

Unless defined otherwise, all technical and scientific terms used herein have the same meaning as commonly understood by those of ordinary skill in the art.

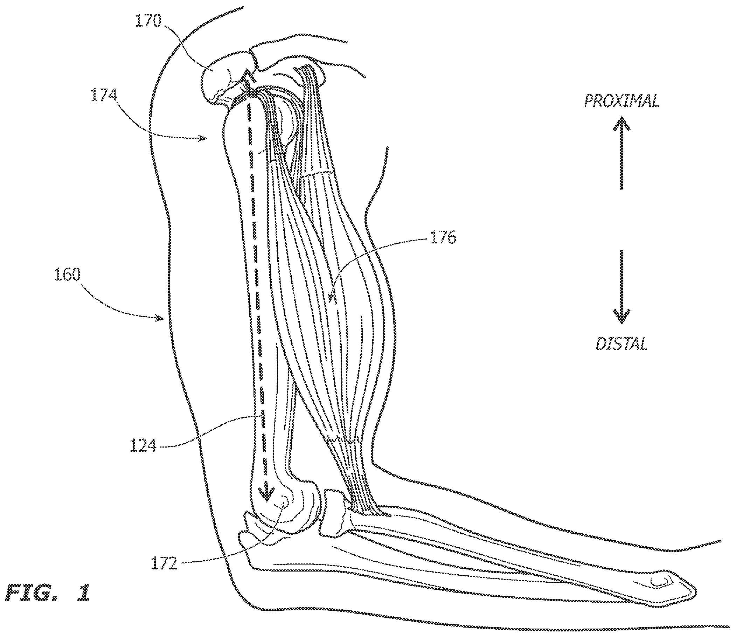

FIG. 1 illustrates a perspective view of an arm including a humeral head. In placing an intraosseous needle within the humeral region of the arm 160 of a patient, a clinician must correctly identify the humeral head 174. This process can be difficult if the patient has regions of thick musculature or excessive adipose tissue 176. In identifying the humeral head 174, the clinician may desire to use anatomical landmarks, to identify the humeral head 174 among the local anatomy of the humeral region. Two such anatomical landmarks may include the acromion 170, located proximally on the arm 160 and the lateral epicondyle 172 located distally on the arm 160. The distance between the acromion 170 and the lateral epicondyle 172 may be considered to be a fixed landmarking distance 124, as the landmarking distance 124 is similar for most patients, providing consistent anatomical landmarks to help locate the humeral head 174.

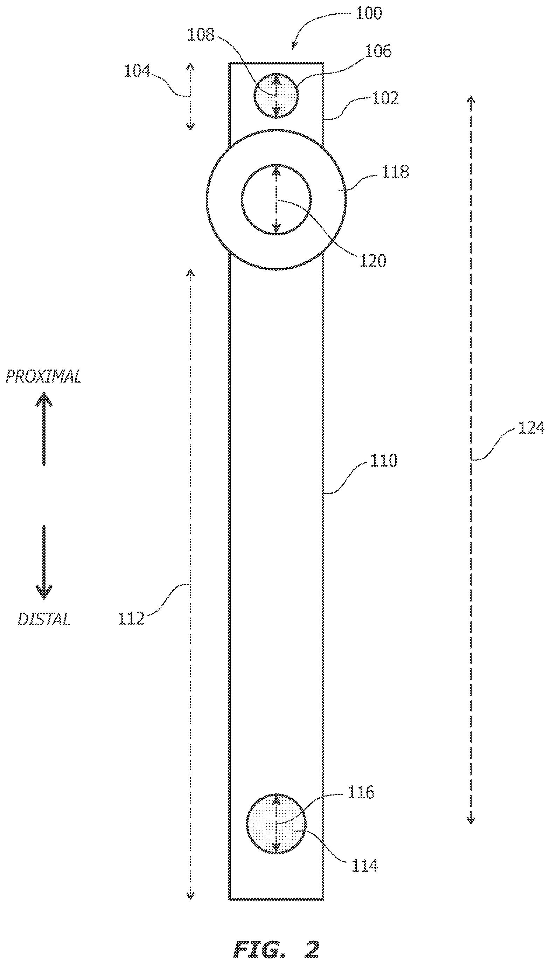

FIG. 2 illustrates a plan view of the intraosseous humeral land marking apparatus ("apparatus") 100, in accordance with some embodiments. In some embodiments, the apparatus 100 may be used to quickly landmark and locate the humeral head for intraosseous needle placement and insertion. In some embodiments, the apparatus 100 includes a tab 102 and a band 110 coupled to an intraosseous needle ring 118. The intraosseous needle ring 118 may include a proximal end, coupled to the tab 102 and a distal end, coupled to the band 110. The intraosseous needle ring 118 may be configured to landmark a humeral head for the placement and insertion of an intraosseous needle when the apparatus 100 is deployed on a patient. The intraosseous needle ring 118 includes an intraosseous needle ring diameter 120. The tab 102 includes an acromion placement circle 106 thereon. The acromion placement circle 106 may be configured to be placed over a patient's acromion during deployment of the apparatus 100. The band 110 includes a lateral epicondyle placement circle ("distal placement circle") 114 thereon. The distal placement circle 114 may be configured to be placed over a patient's lateral epicondyle during deployment of the apparatus 100. In some embodiments, the tab 102 includes a tab length 104 and the band 110 includes a band length 112 wherein the band length 112 is greater than the tab length 104. In some embodiments, the acromion placement circle 106 includes an acromion placement circle diameter 108 and the distal placement circle 114 includes a distal placement circle diameter 116. In some embodiments, the distal placement circle diameter 116 may be less than, greater than or equal to the acromion placement circle diameter 108. In some embodiments, the distal placement circle 114 is located at a distal end of the band 110. The acromion placement circle 106 and the distal placement circle 114 may be separated by a fixed landmarking distance 124, as will be described in more detail herein. Advantageously with the fixed landmarking distance 124 separating the acromion placement circle 106 and the distal placement circle 114, the intraosseous needle ring 118 may be configured to landmark the humeral head while avoiding thick musculature and adipose tissue in the humeral region.

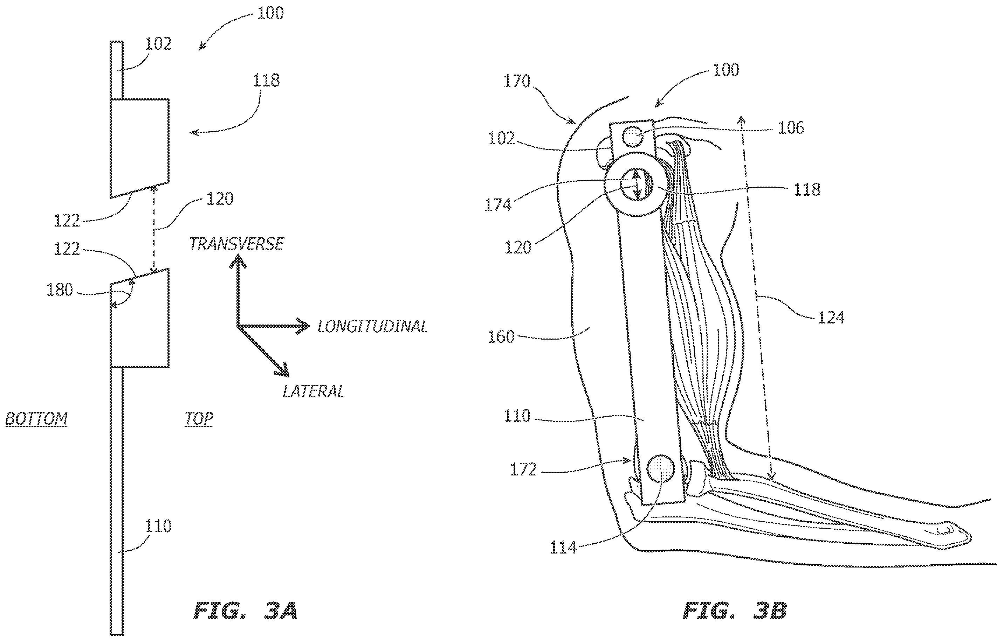

FIG. 3A illustrated a side cross sectional view of the apparatus 100 including the intraosseous needle ring 118, in accordance with some embodiments. In some embodiments, the apparatus 100 includes a top side and a bottom side, wherein when the apparatus is deployed, the bottom side is in physical contact with the arm and the top side is closest to the clinician. In some embodiments, the intraosseous needle ring 118 may protrude longitudinally from the apparatus 100. In some embodiments, the intraosseous needle ring 118 may include a beveled edge 122 to help promote a specific intraosseous needle insertion angle as an intraosseous driver is perpendicular to the arm. For example, the beveled edge 122 may include an angle 180 within the range of 75°-110° in relation to the bottom side, promoting an intraosseous needle insertion angle within the range of 75°-110° in relation to the bottom side. In some embodiments, the intraosseous needle ring 118 may be constructed of the same material as the tab 102 and the band 110. In some embodiments, the intraosseous needle ring 118 may be rigid and constructed of polymers or plastics (e.g., polyethylene terephthalate, polyvinyl chloride, polystyrene, polypropylene or the like).

FIG. 3B illustrates a perspective view of the apparatus 100 on an arm, in accordance with some embodiments. In some embodiments, a portion of or the entire tab 102 may be configured to include an adhesive compound on the bottom side, configured to adhere the tab 102 to an arm 160. In some embodiments, the adhesive compound may be confined to the bottom side of the acromion placement circle 106. In some embodiments, a portion of, or the entire band 110 may be configured to include the adhesive compound on the bottom side, configured to adhere the tab 102 to the arm 160. In some embodiments, the adhesive compound may be confined to the bottom side of the distal placement circle 114. In some embodiments, the apparatus 100 may be constructed of polycarbonate, polypropylene, polyethylene, laminated paper, or the like. In some embodiments, the tab 102 and the band 110 may be configured to include pressure paper. The pressure paper may be configured to change from a first color to a second color with an increase in pressure upon the pressure paper by a hard surface (e.g., humeral head, acromion, lateral epicondyle or the like). In an embodiment, the intraosseous needle ring 118 may include an antimicrobial patch, detachably coupled to the bottom side of the intraosseous needle ring 118 and extending along the intraosseous needle ring diameter 120. In this embodiment, the antimicrobial patch may be configured to target and protect an intraosseous insertion area after the intraosseous needle is inserted therein. In some embodiments, the apparatus 100 may be used as a training device during clinician training to help clinicians quickly familiarize themselves with visual identification of the humeral region and identifying the humeral head.

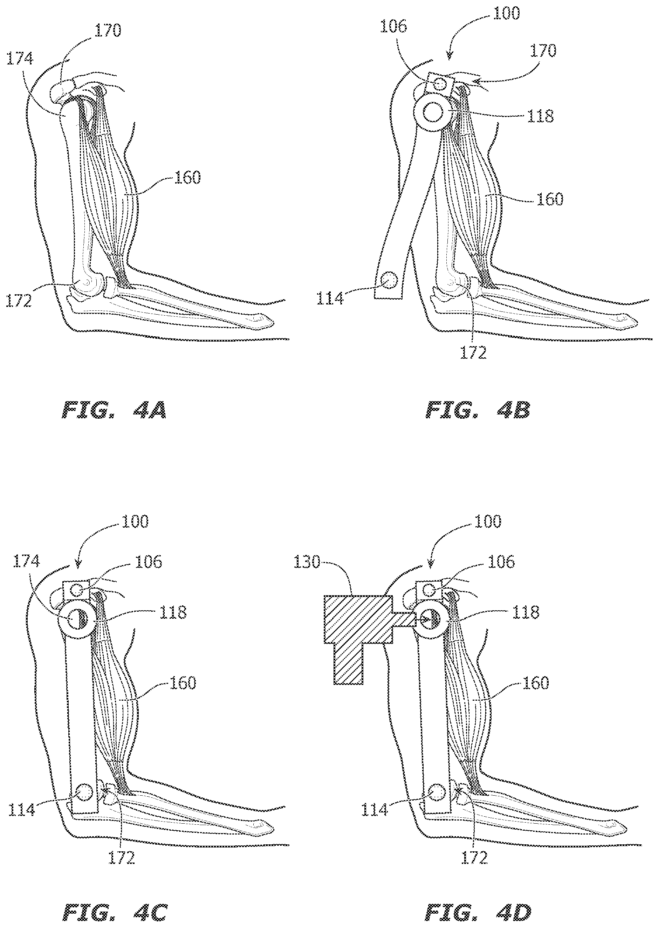

FIGS. 4A-4D illustrate an exemplary method of landmarking the humeral region for intraosseous needle placement, in accordance with some embodiments. In some embodiments, as illustrated in FIG. 4A, the arm 160 of the patient is configured for landmarking by adducting the patient's arm 160 and placing the patient's palm over their umbilicus. Once the arm 160 of the patient is configured for landmarking, the apparatus 100 may be deployed on the patient's arm 160. The acromion placement circle 106 may be placed upon the acromion 170 of the patient, as illustrated in FIG. 4B. With the acromion placement circle 106 placed upon the acromion 170, the distal placement circle 114 may be placed upon the lateral epicondyle 172 as illustrated in FIG. 4C. With the acromion placement circle 106 placed upon the acromion 170 and the distal placement circle 114 placed upon the lateral epicondyle 172, the intraosseous needle ring 118 is configured to indicate the location of the humeral head 174 for intraosseous needle insertion. A intraosseous access system including an intraosseous driver 130 having an intraosseous needle may be assembled for humeral intraosseous access within the intraosseous needle ring 118, as illustrated in FIG. 4D. In some embodiments, a clinician may indicate the proper intraosseous needle area by tracing the intraosseous needle ring 118 with a marking device, before removing the apparatus 100 from the patient's arm 160.

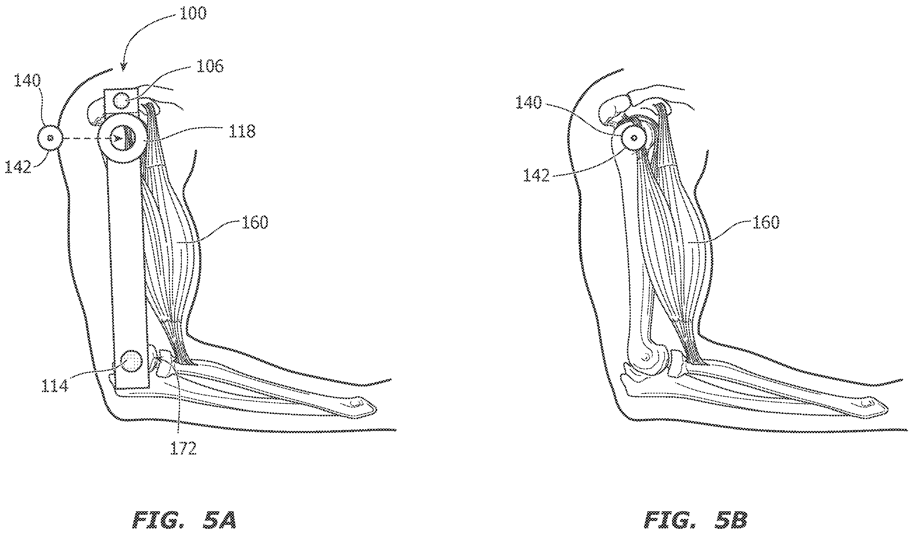

FIGS. 5A-5B illustrate a perspective view of an embodiment of the apparatus 100 in accordance with some embodiments. In this embodiment, as illustrated in FIG. 4A, the apparatus 100 may include a needle stabilizing device 140, configured to fit within the intraosseous needle ring 118, In this embodiment, the bottom side of the needle stabilizing device 140 may be configured to be coupled to the arm 160 by the adhesive compound. In this embodiment, the needle stabilizing device 140 may include a needle stabilizing device lumen 142 therethrough. The needle stabilizing device lumen 142 may be configured to allow and secure an intraosseous needle therethrough. In this embodiment, the needle stabilizing device 140 may indicate the proper location of humeral head 174 and also configure the intraosseous needle at an optimized angle for intraosseous access. In this embodiments, once the needle stabilizing device 140 is coupled to the arm 160, the apparatus 100 may be removed from the arm 160, as illustrated in FIG. 5B. In this embodiment, the needle stabilizing device 140 may be in the shape of a disc, a rectangular prism or the like.

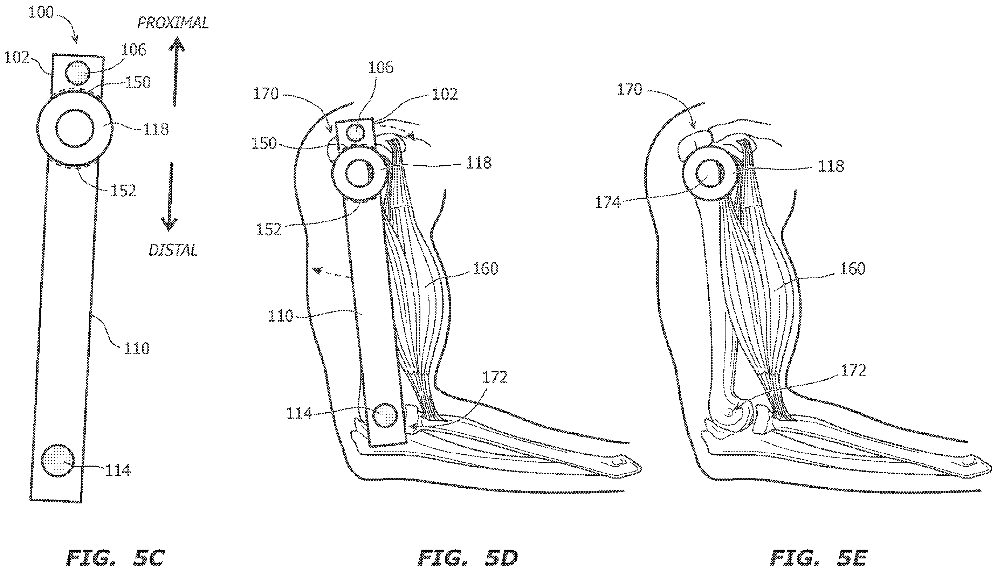

FIGS. 5C-5E illustrate an exemplary method of landmarking the humeral region for proper intraosseous needle placement, in accordance with some embodiments. In an embodiment, as illustrated in FIG. 5C, the tab 102 may include a first score line 150 and the band 110 may include a second score line 152. In this embodiment, a portion of the bottom side of the intraosseous needle ring 118 may include the adhesive compound configured to secure the intraosseous needle ring 118 to the arm 160. In this embodiment, the first score line 150 may be located proximal the intraosseous needle ring 118 and the second score line 152 may be located distal the intraosseous needle ring 118. The apparatus 100 may be placed on the arm 160 wherein the acromion placement circle 106 is over the acromion 170 and the distal placement circle 114 is over the lateral epicondyle 172. The intraosseous needle ring 118 may then be secured to the arm 160, as illustrated in FIG. 5D. In this embodiment, the tab 102 may be configured to separate from the intraosseous needle ring 118 along the first score line 150 and the band 110 may be configured to separate from the intraosseous needle ring 118 along the second score line 152 using a lateral tearing force. As illustrated in FIG. 5E, separating the tab 102 and the band 110 from the intraosseous needle ring 118 leaves the intraosseous needle ring 118 attached to the arm 160, indicating the proper location for intraosseous needle placement in the humeral head 174, as illustrated in FIG. 5E. In this embodiment, the intraosseous needle ring 118 may be configured to stabilize the intraosseous needle, once the intraosseous needle is inserted through the intraosseous needle ring 118.

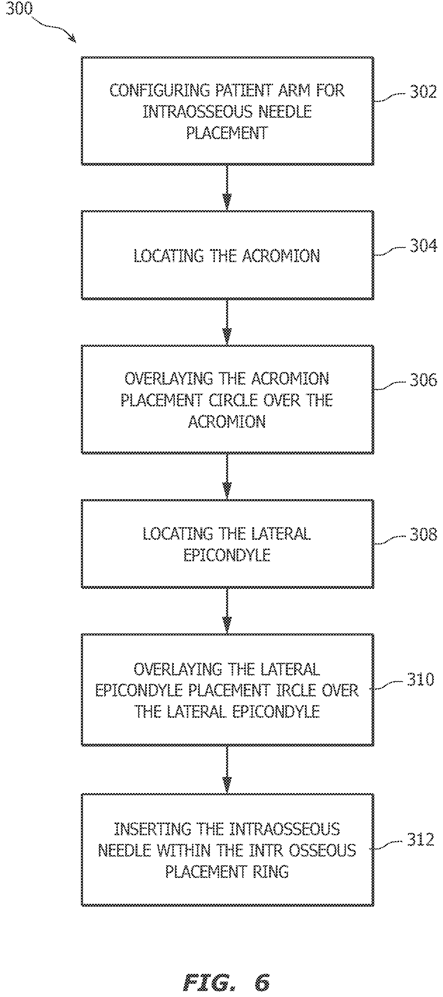

FIG. 6 illustrates a flow chart of the exemplary method 300 of landmarking the humeral region for intraosseous needle placement, in accordance with some embodiments. The method 300 includes configuring the patient's arm for intraosseous needle placement (block 302). In some embodiments, configuring includes a clinician adducting the patient's arm and the patient's palm being placed over the patient's umbilicus. In some embodiments, the method 300 includes locating the acromion 170 (block 304). In some embodiments, the clinician may locate the acromion 170 of the patient using palpation, ultrasound imaging, infrared imaging or the like. The method 300 further includes overlaying the acromion placement circle 106 of the apparatus 100 over the acromion (block 306). In some embodiments, overlaying includes adhering the acromion placement circle 106 to the skin above the acromion 170. The method 300 further includes locating the lateral epicondyle 172 (block 308). In some embodiments, locating includes the clinician locating the lateral epicondyle 172 by palpation, ultrasound imaging, infrared imaging or the like. The method 300 further includes overlaying the distal placement circle 114 over the lateral epicondyle 172 (block 310). In some embodiments, overlaying includes adhering the distal placement circle 114 to the skin above the lateral epicondyle 172. The method 300 includes inserting an intraosseous needle within the intraosseous needle ring 118 (block 312). In some embodiments, inserting includes inserting the intraosseous needle within the intraosseous needle ring 118 into a humeral head 174.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

The invention is defined by the following claims.

## Claims

1. A humeral intraosseous landmarking apparatus (100), comprising:
a tab (102) including an acromion placement circle (106);
a band (110) including a lateral epicondyle placement circle (114), wherein the lateral epicondyle placement circle (114) and the acromion placement circle (106) are separated by a fixed landmarking distance; and
an intraosseous needle ring (118) configured to landmark a humeral head, the intraosseous needle ring (118) having a proximal end coupled to the tab (102) and a distal end coupled to the band (110).

2. The humeral intraosseous landmarking apparatus according to claim 1, wherein the apparatus includes a top side and a bottom side.

3. The humeral intraosseous landmarking apparatus according to any claim of claims 1-2, wherein the tab (102) includes a tab length and the band (110) includes a band length.

4. The humeral intraosseous landmarking apparatus according to claim 3, wherein the band length is greater than the tab length.

5. The humeral intraosseous landmarking apparatus according to any claim of claims 1-4, wherein the lateral epicondyle placement circle (114) is located at a distal end of the band (110).

6. The humeral intraosseous landmarking apparatus according to any claim of claims 2-5, wherein the tab (102), the band (110) and the intraosseous needle ring (118) include an adhesive compound on the bottom side.

7. The humeral intraosseous landmarking apparatus according to any claim of claims 1-6, wherein the intraosseous needle ring (118) includes a beveled edge (122).

8. The humeral intraosseous landmarking apparatus according to claim 7, wherein the beveled edge (122) includes an angle within the range of 75°-110° in relation to the bottom side.

9. The humeral intraosseous landmarking apparatus according to any claim of claims 2-8, wherein the intraosseous needle ring (118) includes an antimicrobial patch detachable coupled to the bottom side of the intraosseous needle ring.

10. The humeral intraosseous landmarking apparatus according to any claim of claims 1-9, wherein the apparatus includes a needle stabilizing device (140) configured to fit within the intraosseous needle ring (118).

11. The humeral intraosseous landmarking apparatus according to claim 10, wherein the needle stabilizing device (140) includes a needle stabilizing device lumen (142) configured to receive therethrough an intraosseous needle and stabilize the intraosseous needle.

12. The humeral intraosseous landmarking apparatus according to any claim of claims 1-11, wherein the tab (102) and the band (110) include pressure paper configured to change from a first color to a second color with an increase in pressure upon the pressure paper by a hard surface.

13. The humeral intraosseous landmarking apparatus according to any claim of claims 1-12, wherein the tab (102) includes a first score line (150) configured to detach the tab (102) from the proximal end of the intraosseous needle ring (118) and the band (110) includes a second score line (152) configured to detach the band (110) from distal end of the intraosseous needle ring (118).

## Patentansprüche

1. Intraossäre Humerus-Landmarkiervorrichtung (100), umfassend:
ein Schild (102) einschließlich eines Akromion-Platzierungskreises (106);
ein Band (110) einschließlich eines lateralen Epicondylus-Platzierungskreises (114), wobei der laterale Epicondylus-Platzierungskreis (114) und der Akromion-Platzierungskreis (106) durch einen festen Markierungsabstand voneinander getrennt sind; und
einen intraossärer Nadelring (118), der konfiguriert ist, um einen Humeruskopf zu markieren, wobei der intraossäre Nadelring (118) ein proximales Ende, das mit dem Schild (102) verbunden ist, und ein distales Ende, das mit dem Band (110) verbunden ist, aufweist.

2. Intraossäre Humerus-Landmarkiervorrichtung nach Anspruch 1, wobei die Vorrichtung eine Oberseite und eine Unterseite einschließt.

3. Intraossäre Humerus-Landmarkiervorrichtung nach einem der Ansprüche 1-2, wobei das Schild (102) eine Schildlänge einschließt und das Band (110) eine Bandlänge einschließt.

4. Intraossäre Humerus-Landmarkiervorrichtung nach Anspruch 3, wobei die Bandlänge größer als die Schildlänge ist.

5. Intraossäre Humerus-Landmarkiervorrichtung nach einem der Ansprüche 1-4, wobei der laterale Epicondylus-Platzierungskreis (114) an einem distalen Ende des Bandes (110) angeordnet ist.

6. Intraossäre Humerus-Landmarkiervorrichtung nach einem der Ansprüche 2-5, wobei das Schild (102), das Band (110) und der intraossäre Nadelring (118) eine Klebeschicht auf der Unterseite einschließt.

7. Intraossäre Humerus-Landmarkiervorrichtung nach einem der Ansprüche 1-6, wobei der intraossäre Nadelring (118) eine abgeschrägte Kante (122) einschließt.

8. Intraossäre Humerus-Landmarkiervorrichtung nach Anspruch 7, wobei die abgeschrägte Kante (122) einen Winkel im Bereich von 75° bis 110° in Bezug auf die Unterseite einschließt.

9. Intraossäre Humerus-Landmarkiervorrichtung nach einem der Ansprüche 2-8, wobei der intraossäre Nadelring (118) ein antimikrobielles Pflaster einschließt, das abnehmbar mit der Unterseite des intraossären Nadelrings verbunden ist.

10. Intraossäre Humerus-Landmarkiervorrichtung nach einem der Ansprüche 1-9, wobei die Vorrichtung eine Nadelstabilisierungseinrichtung (140) einschließt, die konfiguriert ist, um in den intraossären Nadelring (118) zu passen.

11. Intraossäre Humerus-Landmarkiervorrichtung nach Anspruch 10, wobei die Nadelstabilisierungseinrichtung (140) ein Nadelstabilisierungslumen (142) einschließt, das konfiguriert ist, um eine intraossäre Nadel aufzunehmen und die intraossäre Nadel zu stabilisieren.

12. Intraossäre Humerus-Landmarkiervorrichtung nach einem der Ansprüche 1-11, wobei das Schild (102) und das Band (110) druckempfindliches Papier einschließen, das konfiguriert ist, um bei einer Druckerhöhung auf das druckempfindliche Papier durch eine harte Oberfläche von einer ersten Farbe zu einer zweiten Farbe zu wechseln.

13. Intraossäre Humerus-Landmarkiervorrichtung nach einem der Ansprüche 1-12, wobei das Schild (102) eine erste Kerblinie (150) einschließt, die konfiguriert ist, um das Schild (102) vom proximalen Ende des intraossären Nadelrings (118) abzutrennen, und das Band (110) eine zweite Kerblinie (152) einschließt, die konfiguriert ist, um das Band (110) vom distalen Ende des intraossären Nadelrings (118) abzutrennen.

## Revendications

1. Appareil de marquage intra-osseux huméral (100), comprenant :
une languette (102) incluant un cercle de placement d'acromion (106) ;
une bande (110) incluant un cercle de placement d'épicondyle latéral (114), dans laquelle le cercle de placement d'épicondyle latéral (114) et le cercle de placement d'acromion (106) sont séparés par une distance fixe de marquage ; et
une bague à aiguille intra-osseuse (118) configurée pour marquer une tête humérale, la bague à aiguille intra-osseuse (118) présentant une extrémité proximale couplée à la languette (102) et une extrémité distale couplée à la bande (110).

2. Appareil de marquage intra-osseux huméral selon la revendication 1, dans lequel l'appareil inclut un côté supérieur et un côté inférieur.

3. Appareil de marquage intra-osseux huméral selon une quelconque revendication des revendications 1-2, dans lequel la languette (102) inclut une longueur de languette et la bande (110) inclut une longueur de bande.

4. Appareil de marquage intra-osseux huméral selon la revendication 3, dans lequel la longueur de bande est supérieure à la longueur de languette.

5. Appareil de marquage intra-osseux huméral selon une quelconque revendication des revendications 1-4, dans lequel le cercle de placement d'épicondyle latéral (114) est situé à une extrémité distale de la bande (110).

6. Appareil de marquage intra-osseux huméral selon une quelconque revendication des revendications 2-5, dans lequel la languette (102), la bande (110) et la bague à aiguille intra-osseuse (118) incluent un composé adhésif sur le côté inférieur.

7. Appareil de marquage intra-osseux huméral selon une quelconque revendication des revendications 1-6, dans lequel la bague à aiguille intra-osseuse (118) inclut un bord biseauté (122).

8. Appareil de marquage intra-osseux huméral selon la revendication 7, dans lequel le bord biseauté (122) inclut un angle à l'intérieur de la plage de 75°-110° par rapport au côté inférieur.

9. Appareil de marquage intra-osseux huméral selon une quelconque revendication des revendications 2-8, dans lequel la bague à aiguille intra-osseuse (118) inclut un timbre antimicrobien détachable couplé au côté inférieur de la bague à aiguille intra-osseuse.

10. Appareil de marquage intra-osseux huméral selon une quelconque revendication des revendications 1-9, dans lequel l'appareil inclut un dispositif de stabilisation d'aiguille (140) configuré pour s'adapter à l'intérieur de la bague à aiguille intra-osseuse (118).

11. Appareil de marquage intra-osseux huméral selon la revendication 10, dans lequel le dispositif de stabilisation d'aiguille (140) inclut une lumière de dispositif de stabilisation d'aiguille (142) configurée pour recevoir à travers celui-ci une aiguille intra-osseuse et stabiliser l'aiguille intra-osseuse.

12. Appareil de marquage intra-osseux huméral selon une quelconque revendication des revendications 1-11, dans lequel la languette (102) et la bande (110) incluent un papier à pression configuré pour changer d'une première couleur à une seconde couleur avec une augmentation de pression sur le papier à pression par une surface dure.

13. Appareil de marquage intra-osseux huméral selon une quelconque revendication des revendications 1-12, dans lequel la languette (102) inclut une première ligne entaillée (150) configurée pour détacher la languette (102) de l'extrémité proximale de la bague à aiguille intra-osseuse (118) et la bande (110) inclut une seconde ligne entaillée (152) configurée pour détacher la bande (110) de l'extrémité distale de la bague à aiguille intra-osseuse (118).
